# EUROPEAN PATENT APPLICATION

(11) **EP 1 630 164 A1**
(43) Date of publication of application: **01.03.2006**
(21) Application number: 04425644.4
(22) Date of filing: 31.08.2004
(51) Int. Cl.: C07D 487/04, A61K 31/519, A61P 15/00, A61P 9/00, C07D 239/00, C07D 231/00

(54) **Pyrazolopyrimidinones as phosphodiesterase inhibitors**

(71) Applicant: Expotrend Company SA, 6901 Lugano (CH)
(72) Inventor: Sparatore, Anna, 20146 Milan (IT); Wallace, John L., Cochrane, Alberta T4C IT6 (CA)
(74) Representative: Parisi, Luigi

(57) **Abstract**

Compounds of formula 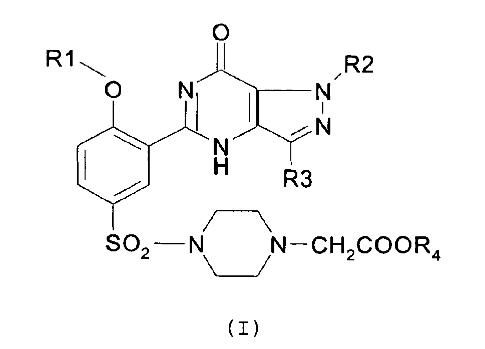 wherein
R1 is H, C₁-C₃-alkyl, C₃-C₅-cycloalkyl or C₁-C₃-perfluoroalkyl,R2 is H, C₁-C₆-alkyl optionally substituted by OH, C₁-C₃-alkoxy or C₁-C₃-perfluoroalkyl, R3 is C₁-C₆-alkyl, C₃-C₆-alkenyl, C₃-C₆-alkinyl, C₃-C₇-cycloalkyl, C₁-C₆-perfluoroalkyl or C₃-C₆-cycloalkyl-C₁-C₆-alkyl, R₄ = H, ADT-OH, cysteine, agmatine, arginine, aminoguanidine or agents releasing or stimulating the release of hydrogen sulfide, and salts thereof. The compounds are suitable for treating impotence, cardiovascular disorders, diseases characterized by disorders of gut motility, gastropathy of diabetic and nondiabetic origin, in general diseases where cytoprotection is important.

## Description

### Background of the invention

It is known that cAMP and cGMP mediate biological responses initiated by diverse extracellular signals. By catalyzing hydrolysis of the 3-5-phosphodiester bond of cyclic nucleotides, cyclic nucleotide phosphodiesterases (PDEs) regulate intracellular concentrations and the effects of these second messengers. Phosphodiesterases (PDEs) are a family of enzymes involved in regulating intracellular signaling. PDEs are the intracellular enzymes responsible for the degradation of both cyclic AMP (cAMP) and cyclic GMP (cGMP). To date, 11 PDE groups are known and these can be further differentiated into 21 sub-groups.

PDEs include a large group of structurally related enzymes that differ in their primary structures, affinities for cAMP and cGMP, responses to specific effectors, sensitivities to specific inhibitors, and mechanisms of regulation.

Most families are comprised of more than one gene; 14 different PDE genes have been identified. Within different families, tissue-specific mRNAs are generated from the same gene by the use of different transcription initiation sites or by alternative mRNA splicing.

The widely divergent N-terminal portions of PDEs contain determinants that confer regulatory properties specific to the different gene families, e.g. calmodulin-binding domains (PDE1); two non-catalytic cyclic nucleotide-binding domains (PDEs 2, 5, and 6); N-terminal membrane-targeting (PDE4) or hydrophobic membrane-association (PDE3) domains; and calmodulin (PDE1)-, cyclic AMP (PDEs 1, 3, and 4)-, and cGMP (PDE5)-dependent protein kinase phosphorylation sites, etc.

Most cells contain representatives of several PDE families in different amounts, proportions, and subcellular locations. In some instances, a specific PDE regulates a unique cellular function, e.g. photoreceptor PDE6 in cGMP-dependent initiation of visual transduction. In individual cells, different PDEs, with their different responses to regulatory signals, participate in integrating multiple inputs in the complex modulation and termination of cyclic nucleotide signals and responses, e.g. their magnitude and duration, their functional and spatial compartmentation, and their attenuation by short-term feedback or long-term desensitization.

The most significant factor in the regulation of PDEs and their role in the cyclic nucleotide metabolism, is the fact that PDE isoenzymes and families tend to be very tissue-specific. The heterogeneity in PDEs distribution, combined with the existence of drugs designed to specific PDE types, has generated considerable interest for their clinical use.

There is a large body of evidence that in general GMP-mediated physiological processes relate to smooth muscle relaxation, neurotransmission, inhibition of platelet aggregation and immune response. As example non limitative, the use of PDEs inhibitors, and particularly PDE5 inhibitors, like sildenafil, on smooth muscle dysfunction has been widely described.

Sildenafil has proven to be effective in the therapy of male erectile dysfunction. Small doses of sildenafil may be a useful combination to inhaled iloprost in the management of pulmonary hypertension. In female sexual dysfunction and infertility, genital blood flow and endometrial thickening are enhanced after application of the compound. In gastrointestinal disorders, sildenafil also exerts several effects that might be of clinical relevance. In patients with heart failure, endothelial dysfunction is influenced by the phosphodiesterase-5 (PDE 5) inhibitor and exercise capacity might be improved. Moreover, in the treatment of Raynaud's phenomenon, a disease without highly effective medical treatment option yet, first observations with sildenafil seem to be promising.

Disorders such as erectile dysfunction (ED) and female sexual dysfunction are becoming increasingly more important as a result of the aging population. Sexual dysfunction is often associated with disorders such as diabetes, hypertension, coronary artery disease, neurological disorders, and depression. In some patients, sexual dysfunction may be the presenting symptom of such disorders.

Andersson K.E., Pharmacology of penile erection, Pharmacol Rev., Sept. 2001; 53 (3) 417-450, summarized some of the information related to the pathways involved in erectile function. The degree of contraction of corpus cavernosal smooth muscle determines the functional state of the penis. The balance between contraction and relaxation is controlled by central and peripheral factors that involve many transmitters and transmitter systems. At the cellular level, smooth muscle relaxation occurs following the release of acetylcholine from the parasympathetic nerves.

The nerves and endothelium of sinusoids and vessels in the penis produce and release transmitters and modulators that control the contractile state of corporal smooth muscles. Although the membrane receptors play an important role, downstream signaling pathways are also important.

The nitric oxide (NO) pathway is of critical importance in the physiologic induction of erection. The drugs currently used to treat erectile dysfunction were developed as a result of experimental and clinical work that demonstrated that NO released from nerve endings relaxes the vascular and corporal smooth muscle cells of the penile arteries and trabeculae, resulting in an erection.

Recently, also other uses than impotence have been suggested on the basis of experimental evidence (Cremers B, Bohm M., Non erectile dysfunction application of sildenafil. Herz. 2003 Jun; 28(4):325-33).

NO is produced by the enzyme nitric oxide synthase (NOS). Three forms have been identified: nNOS, eNOS, and iNOS, which are produced by the genes *NOS1* (nNOS); *NOS2* (iNOS), and *NOS3* (eNOS). This nomenclature is derived from the source of the original isolates. nNOS was found in neuronal tissue, iNOS was found in immunoactivated macrophage cell lines, and eNOS was found in vascular endothelium. All forms of NOS produce NO, but a variety of factors trigger and regulate this process. NOS plays many roles, ranging from homeostasis to immune system regulation. These subtypes are not limited to the tissues from which they were first isolated. Each NOS subtype may play a different biological role in various tissues.

Now, nNOS and eNOS are considered mostly constitutive forms because they share biochemical features. They are calcium-dependent, they require calmodulin and reduced nicotinamide adenine dinucleotide phosphate for catalytic activity, and they are competitively inhibited by arginine derivatives. These two subtypes use the biochemical pathway that targets cyclic guanosine monophosphate (cGMP). They are involved in the regulation of neurotransmission and blood flow, respectively. iNOS is considered mostly inducible because it is calcium-independent. iNOS is induced by the inflammatory process, in which it is involved in the production nitrogenous amines. This subtype has been shown to be involved in the carcinogenic process, leading to transitional cell carcinoma.

All three NOS subtypes produce NO by oxidation of L-arginine, which is one of the basic amino acids. It circulates in the blood and is found in cells synthesized from the urea cycle or from oral ingestion. The concentration of L-arginine within the cell far exceeds that in the circulation. Inside the cell, NOS catalyzes the oxidation of L-arginine to NO and L-citrulline. Endogenous blockers of this pathway have been identified. The gaseous NO that is produced, acts as a neurotransmitter or paracrine messenger. Its biologic half-life is of only 5 seconds. NO may act within the cell or diffuse and interact with nearby target cells.

Potential ways to alter NO levels include the following: directly administering NO as a gas; administering NO donors such as nitrates, nitrites, and inorganic nitroso compounds; administering of NO agonists such as ACE, which enhances the production of NO within endothelial cells; preserving cGMP phosphodiesterase, which primarily hydrolyze cGMP type 5, provided the basis for the development of sildenafil, vardenafil, and tadalafil.

### Field of the invention

The present invention relates to new pyrazolo[4,3-d]pyririmidin-7-ones compounds that are selective PDEs inhibitors of cyclic guanosine 3',5'-monophosphate phosphodiesterases (cGMP PDE) having utility in a variety of therapeutic areas including impotence; cardiovascular disorders such as angina, hypertension, hearth failure and atherosclerosis; disease characterized by disorders of gut motility e.g. irritable bowel syndrome (IBS); gastropathy of diabetic and nondiabetic origin; in general diseases where cytoprotection is important, e.g. ulcer healing and prevention,etc.

The compounds of the present invention are potent inhibitors of (cGMP PDEs) in contrast to their inhibition of (cAMP). This selective enzyme inhibition leads to elevated cGMP levels that are the basis for their pharmacological properties.

Differently from the classical 5PDEs inhibitors, such as sildenafil, that are poorly effective in conditions characterized by mild to severe endothelium dysfunction, the compounds of the present invention are very effective in subjects at risk, such as diabetic, smokers, aging people, patients affected by chronic debilitating diseases but also better tolerated and with less side effects related to excessive nitric oxide production (for example cardiopathic and angina patients and particularly those requiring the chronic use of transdermal nitrates). And, furthermore, in view of this very positive profile, these agents represent not only a symptomatic remedy but also a causative treatment.

Other agents are known to induce smooth relaxation. Transcription factor inhibitors represent one large class of agents. Among them, hydrogen sulfide releasing agents can be considered. Hydrogen sulfide has been reported to work synergistically with nitric oxide, but the effects on impotence although not definitive are someway contradictory [(Environ Health Perspect., 1998 Sep;106(9):611-3). Multiple system atrophy following chronic carbon disulfide exposure. Frumkin H.; Arch Environ Health. 1994 Jul-Aug;49(4):273-8. Epidemiological study of the effects of carbon disulfide on male sexuality and reproduction. Vanhoorne M, Comhaire F, De Bacquer D.]

Surprisingly, we have found that the compounds of the present invention that are able to donate, transfer or release hydrogen sulfide (H₂S) or stimulate the endogenous synthesis of hydrogen sulfide, do exert marked pharmacological effects.

Substances releasing or stimulating the release of hydrogen sulfide that can be linked directly or indirectly via bifunctional groups are N-acetylpenicillamine, bucillamine, carbocysteine, cysteamine, cysteine, cystathionine, homocysteine, mecysteine, methionine, pantetheine, penicillamine, penicillamine disulfide, thioacetic acid, thiodiglycolic acid, thioglycolic acid, thiolactic acid, 2-thiolhistidine, thiomalic acid, thioctic acid, thiosalicylic acid, tiopronin. Other substances releasing and/or stimulating the release of hydrogen sulfide that can be linked to drugs are 5-(p-hydroxyphenyl)-3H-1,2-dithiol-3-thione (ADT-OH), 1,3-dithiol-2-thione-5-carboxylic acid, 3-hioxo-3H-1,2-dithiole-5-carboxylic acid, 3-thioxo-3H-1,2-dithiole-4 carboxylic acid.

The compounds of the present invention have the general formula: 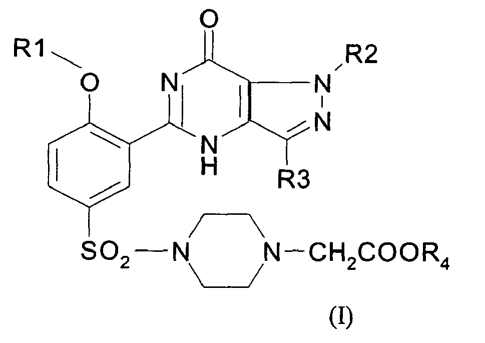 wherein
R1 is H, C₁-C₃-alkyl, C₃-C₅-cycloalkyl or C₁-C₃₋perfluoroalkyl,
R2 is H, C₁-C₆-alkyl optionally substituted by OH, C₁-C₃₋alkoxy or C₁-C₃-perfluoroalkyl,
R3 is C₁-C₆-alkyl, C₃-C₆-alkenyl, C₃-C₆-alkinyl, C₃-C₇₋cycloalkyl, C₁-C₆-perfluoroalkyl or C₃-C₆-cycloalkyl-C₁-C₆₋alkyl,
R₄ = ADT-OH, cysteine, agmatine, arginine, aminoguanidine, or agents releasing or stimulating the release of hydrogen sulfide, and salts thereof.

When the compounds include at least one asymmetric carbon atom, the products can be used in racemic mixture or in form of single enantiomer.

The invention relates also to the use of a cGMP PDE inhibitor or a pharmaceutically acceptable salt thereof and to pharmaceutical compositions containing either entity.

The compounds of the present invention can be salified with pharmaceutically acceptable acids such as citric acid, fumaric acid, maleic acid etc. Preferred salts are lipoate salts with thioctic acid (as racemic or with its enantiomers) and the nitrate salts.

When R₄ is H or when the R₄ has acidic properties, the preferred salt is agmatine salt.

The nitrate salts can be obtained reacting the compounds of formula (I) with nitric acid or silver nitrate according to methods well known in the art.

The compounds of the present invention can be administered in the form of any pharmaceutical formulation, the nature of which will depend upon the route of administration and the nature of the disease to be treated. These pharmaceutical compositions can be prepared by conventional methods, using compatible, pharmaceutically acceptable excipients or vehicles. Examples of such compositions include capsules, tablets, syrups, powders and granulates for the preparation of extemporaneous solutions, injectable preparations, rectal, nasal, ocular, vaginal etc. A preferred route of administration is the oral route.

A further object of the present invention is a method for treating impotence, cardiovascular disorders, diseases characterized by disorders of gut motility, gastropathy of diabetic and nondiabetic origin, in general diseases where cytoprotection is important, in particular a method for treating angina, hypertension, hearth failure and atherosclerosis, irritable bowel syndrome (IBS), ulcer healing and prevention, comprising administering the compounds of the present invention or salts thereof to a subject in need thereof.

The following non-limitative examples further describe and enable an ordinary skilled in the art to make and use the invention.

### EXAMPLE 1. Synthesis of sildenafil analog ester with 5-(p-hydroxyphenyl)-3H-1,2-dithiol-3-thione.

The synthesis of 5-(2-ethoxy-5-4-(hydroxycarbonylmethyl)piperazinylsulfonyl)-phenyl-1-methyl-3-n-propyl-1, 6-dihydro-7H-pyrazolo[4,3-d]pyrimidin-7-one was performed according to Bell, US pat. 5,346,901 and Kim in Biorg. Med. Chem.(2001) 9 3013-3021.

The sildenafil analog ester was prepared employing dicyclohexylcarbodiimide (DCC) as coupling agent in presence of dimethylaminopyridine. The solvent used was methylene chloride purified to remove traces of ethanol. In a 50 ml flask 0.193 mmol of sildenafil analog 5-(2-ethoxy-5-(4-hydroxycarbonylmethyl)piperazinylsulfonyl)-phenyl)-1-methyl-3-n-propyl-1,6-dihydro-7H-pyrazolo[4,3-d]pyrimidin-7-one were charged with 0,201 mmol of 5-(p-hydroxyphenyl)-3H-1,2-dithiol-3-thione as well as 1 mg of dimethylaminopyridine (DMAP), and 51,4 mg of dicyclohexylcarbodiimide (DCC) in 10 ml of methylene chloride. The mixture was stirred for 30 minutes at room temperature. At the end of the reaction after filtration, the solution is extracted with NaOH 0.1N and water. After removal of the solvent the product is cromatographed on silica gel with dichloromethane and 1% methanol and crystallized by dichloromethane: melting point 188-189°C. Anal. calcd. C₃₂H₃₄N₆O₆S₄ C% 52.87; H% 4.71; N% 11.56; S% 17.64; found C% 52.54; H% 4.74; N% 11.40; S% 17.81.

### EXAMPLE 2. Synthesis of sildenafil analog amide with cysteine.

The synthesis of 5-(2-ethoxy-5-(4-(hydroxycarbonylmethyl)piperazinylsulfonyl)-phenyl)-1-methyl-3-n-propyl-1,6-dihydro-7H-pyrazol[4,3-d]pyrimidin-7-one was performed according to what reported by Bell in US pat. 5,346,901 and by Kim in Biorg. Med. Chem. (2001) 9 3013-3021.

The first step is the preparation of the ter-butyl-L-(S-trityl)cysteine.

2.83 mmol of S-tritylcysteine, 18 ml of terbutylacetate and 0.27 ml of HClO₄ (70%) were introduced in a 50 ml flask. The reaction was carried out at room temperature for 24 hours under nitrogen athmosphere. To the mixture, 20 ml of ethyl acetate and NaHCO₃ 1M up to pH 8 were added. The precipitate was filtered and the organic phase was separated and extracted with HCl 0.5 N, dried on sodium sulphate and evaporated. The product was cromatographed on silica gel using CH₂Cl₂ with 1% methanol as eluting solvent.

In a 100 ml flask 0.9 mmol of sildenafil analog 5-(2-ethoxy-5-(4-hydroxycarbonylmethyl)piperazinylsulfonyl)-phenyl)-1-methyl-3-n-propyl-1,6-dihydro-7H-pyrazolo[4,3-d]pyrimidin-7-one were charged with 0,9 eq. of ter-butyl-L-(S-trityl)cysteine as well as 1,5 eq. of butanol dissolved in 45 ml of CH₂Cl₂. The reaction mixture was cooled at 0 C and 1.1 eq. of dicyclohexylcarbodiimide (DCC) with 2 eq. of N-methylmorpholine were added. The mixture was stirred for 5 hours at room temperature under nitrogen. At the end of the reaction after filtration and removal of the solvent, the product was cromatographed on silica gel with dichloromethane and 1% methanol and then washed with petrol ether.

At a solution of 0.608 mmol of this product in 12 ml of CH₂Cl₂, 2 mmol of triethylsilane and 10 ml of trifluoroacetic acid were added and the mixture was stirred at room temperature and under nitrogen athmosphere for 4 hours. The reaction was maintained at room temperature for further 4 hours. After evaporation of CH₂Cl₂ and trifluoroacetic acid, the solid residue was washed with ether and recrystalized with ethylacetate (melting point 166-168 °C). Anal. calcd. C₂₆H₃₅N₇O₇S₂·CF₃COOH·H₂O C% 44.61; H% 5.08; N% 13.01; S% 8.51; found C% 44.99; H% 4.96; N% 12.82; S% 8.49.

### Example 3. Salt of α-lipoic acid with sildenafil ester of Example 1.

A solution of 1 mmol of compound of Example 1 in 25 ml of dichloromethane was added to 1 mmol of α-lipoic acid in 25 ml of dichloromethane. The solution was evaporated and the residue was washed with ether and isolated.

### Example 4. Biological data

The compounds of the present invention have been tested in vitro and found to be potent and selective inhibitors of cGMP PDE5. For example the compounds of Examples 1 and 2 have an IC50 = 6.8-7.2 nM vs PDE5 enzyme, but only a weak inhibitory activity against PDE2 and PDE3 enzymes with an IC50 >50 mM.

## Claims

1. Compounds of formula 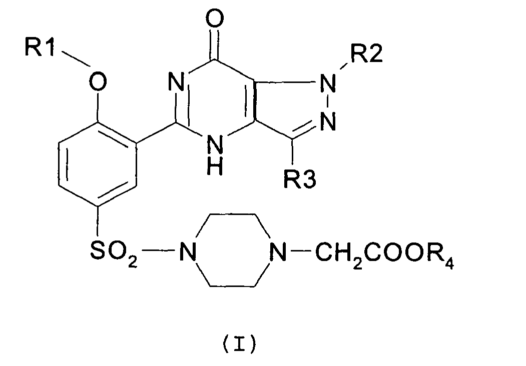 wherein
R1 is H, C₁-C₃-alkyl, C₃-C₅-cycloalkyl or C₁-C₃₋perfluoroalkyl,R2 is H, C₁-C₆-alkyl optionally substituted by OH, C₁-C₃-alkoxy or C₁-C₃-perfluoroalkyl,R3 is C₁-C₆₋alkyl, C₃-C₆-alkenyl, C₃-C₆-alkinyl, C₃-C₇-cycloalkyl, C₁₋C₆-perfluoroalkyl or C₃-C₆-cycloalkyl-C₁-C₆-alkyl, R₄ = H, ADT-OH, cysteine, agmatine, arginine, aminoguanidine or agents releasing or stimulating the release of hydrogen sulfide, and salts thereof.

2. Compounds according to claim 1, wherein the agents releasing or stimulating the release of hydrogen sulfide are selected from the group consisting of N-acetylpenicillamine, bucillamine, carbocysteine, cysteamine, cysteine, cystathionine, homocysteine, mecysteine, methionine, pantetheine, penicillamine, penicillamine disulfide, thioacetic acid, thiodiglycolic acid, thioglycolic acid, thiolactic acid, 2-thiolhistidine, thiomalic acid, thioctic acid, thiosalicylic acid, tiopronin, 5-(p-hydroxyphenyl)-3H-1,2-dithiol-3-thione, 1,3-dithiol-2-thione-5-carboxylic acid, 3-thioxo-3H-1,2-dithiole-5-carboxylic acid, 3-thioxo-3H-1,2-dithiole-4 carboxylic acid.

3. Compound according to claim 1, that is sildenafil analog ester with 5-(p-hydroxyphenyl)-3H-1,2-dithiol-3-thione.

4. Compound according to claim 1, that is sildenafil analog amide with cysteine.

5. Salts of compounds according to claim 1, **characterized in that** they are the nitrate salts.

6. Compounds according to claim 1, **characterized in that** they are salified with thioctic acid.

7. Compounds according to claim 1, **characterized in that** when R₄ is H, the salt is agmatine or arginine salt.

8. Pharmaceutical compositions containing a compound of formula (I) as active ingredient and pharmaceutically acceptable adjuvants or carriers.

9. Use of a compound as claimed in one of the claims 1-7 for the manufacture of a medicament for treatment of impotence, cardiovascular disorders, diseases **characterized by** disorders of gut motility, gastropathy of diabetic and nondiabetic origin, in general diseases where cytoprotection is important.

10. Use of a compound as claimed in one of the claims 1-7 for the manufacture of a medicament for treatment of angina, hypertension, hearth failure and atherosclerosis, irritable bowel syndrome (IBS), ulcer healing and prevention.
